# EUROPEAN PATENT APPLICATION

(11) **EP 3 842 002 A1**
(43) Date of publication of application: **30.06.2021**
(21) Application number: 19219699.6
(22) Date of filing: 24.12.2019
(51) Int. Cl.: A61B 18/20, A61B 90/00, A61N 5/06, A61B 34/10, A61B 18/00

(54) **PROVIDING FEEDBACK ON A TREATMENT OPERATION PERFORMED ON A BODY PART OF A SUBJECT**

(71) Applicant: Koninklijke Philips N.V., 5656 AG Eindhoven (NL)
(72) Inventor: Fernando, Shakith Devinda, 5656 AE Eindhoven (NL); Brouwer, Jan, 5656 AE Eindhoven (NL); Van Bree, Karl Catharina, 5656 AE Eindhoven (NL); Zeitouny, Mounir, 5656 AE Eindhoven (NL); De Bruijn, Frederik Jan, 5656 AE Eindhoven (NL)
(74) Representative: Philips Intellectual Property & Standards

(57) **Abstract**

According to an aspect, there is provided an apparatus for use with a treatment device for providing feedback to a user on a treatment operation performed on a body part of a subject, wherein the treatment device is configured to apply light pulses to skin of the body part to perform the treatment operation, wherein a light pulse applied to the skin treats an area of the skin. The apparatus comprises a processing unit configured to receive a first measurement signal from a first sensor, the first measurement signal comprising information about positions and/or movements of the treatment device over time; for a light pulse previously applied by the treatment device to the body part during the treatment operation, process the first measurement signal to estimate a previous treatment position as a position of the treatment device relative to the body part when the light pulse was generated; for the previously applied light pulse, estimate a previous treatment area for the light pulse corresponding to the area of skin of the body part that the light pulse was applied to when the treatment device was at the previous treatment position; process the first measurement signal to estimate a current position of the treatment device relative to the body part; estimate a current treatment area corresponding to an area of skin that the treatment device would apply a light pulse to while in the current position; and generate a feedback control signal for a feedback unit, wherein the feedback control signal is configured to cause the feedback unit to generate feedback indicating whether the current treatment area corresponds, or substantially corresponds, to a previous treatment area.

## Description

### FIELD OF THE INVENTION

This disclosure relates to providing feedback on a treatment operation performed on a body part of a subject, and in particular to providing feedback on a treatment operation in which light pulses are applied to skin on the body part.

### BACKGROUND OF THE INVENTION

Techniques for removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. Home-use photoepilation devices, for example the Philips Lumea device, use intense pulsed light (IPL) from high intensity light sources, e.g. Xenon flash lamps that produce high output bursts of broad spectrum light.

A photoepilation treatment is characterized by the user of the photoepilation device treating relatively small areas of the skin for the purpose of hair removal. The photoepilation treatment uses intense light to heat melanin in hair and hair roots, which puts the hair follicles into a resting phase, preventing hair re-growth. For effective use of this technology for hair removal, the user must treat the skin completely without leaving any gaps. Since this effect is only of limited duration, treatment has to be repeated on a regular basis: typically once every 4 to 8 weeks in the maintenance phase after an initial period of about two months in which treatment is performed once every two weeks.

In a typical photoepilation treatment, the user of the photoepilation device must repeatedly manually position the photoepilation device on the skin and trigger a light pulse in order to cover a full body part (e.g. an arm, a leg, etc.). However, as a photoepilation device typically does not provide any feedback to the user about the areas that have already been treated, and there are little or no user-perceptible changes to the skin or hairs on applying a light pulse or shortly after applying a light pulse, it is difficult for a user to achieve complete coverage of a body part and/or avoid over-treating certain areas of the body part. In addition, some types of treatment device can make use of different attachments for treating different parts of the body, and these attachments can change the size of the area of skin treated with each light pulse. This makes it more difficult for a user to determine which parts of the skin have been treated.

Therefore it is desirable to be able to determine and provide feedback on a treatment operation performed on a body part of a subject to a user of the treatment device.

### SUMMARY OF THE INVENTION

According to a first specific aspect, there is provided an apparatus for use with a treatment device for providing feedback to a user on a treatment operation performed on a body part of a subject, wherein the treatment device is configured to apply light pulses to skin of the body part to perform the treatment operation, wherein a light pulse applied to the skin treats an area of the skin. The apparatus comprises a processing unit configured to receive a first measurement signal from a first sensor, the first measurement signal comprising information about positions and/or movements of the treatment device over time; for a light pulse previously applied by the treatment device to the body part during the treatment operation, process the first measurement signal to estimate a previous treatment position as a position of the treatment device relative to the body part when the light pulse was generated; for the previously applied light pulse, estimate a previous treatment area for the light pulse corresponding to the area of skin of the body part that the light pulse was applied to when the treatment device was at the previous treatment position; process the first measurement signal to estimate a current position of the treatment device relative to the body part; estimate a current treatment area corresponding to an area of skin that the treatment device would apply a light pulse to while in the current position; and generate a feedback control signal for a feedback unit, wherein the feedback control signal is configured to cause the feedback unit to generate feedback indicating whether the current treatment area corresponds, or substantially corresponds, to a previous treatment area.

In some embodiments the first sensor is one of: a movement sensor, an accelerometer, a gyroscope, a magnetometer, an air pressure sensor, and an imaging unit.

In some embodiments the sensor is an imaging unit, the first measurement signal is a plurality of images or a video sequence, and the plurality of images or the video sequence is processed to identify positions and/or movements of the treatment device over time. In these embodiments the processing unit can be configured to: identify skin features and/or body part features in the images or video sequence; monitor movement of those skin features and/or body part features in the images or video sequence over time; and determine positions and/or movements of the treatment device relative to the body part from the movement of those skin features and/or body part features in the images or video sequence. In these embodiments the processing unit may be further configured to receive a second measurement signal from a movement sensor, the second measurement signal comprising information about positions and/or movements of the treatment device over time; and the processing unit can be configured to process the first measurement signal and the second measurement signal to estimate the previous treatment positions and the current position.

In some embodiments the feedback control signal is a display control signal, and the feedback unit is a display unit. In these embodiments the display control signal can be configured to cause the display unit to generate a graphical display comprising graphical representations of the previous treatment areas and the current treatment area overlaid on the body part. In these embodiments the display unit may be a display screen and the display control signal may include a plurality of images or a video sequence of the body part on which the graphical representations are overlaid. In alternative embodiments, the display unit may be a projector and the display control signal may be configured to cause the projector to project the graphical representations onto the body part of the subject according to the estimated previous treatment areas and the current treatment area. In other alternative embodiments, the display unit may be configured to display the graphical representations as part of an augmented reality display of the body part.

In some embodiments the processing unit is configured to estimate the previous treatment areas and estimate the current treatment area based on: (i) a size of an aperture in the treatment device through which the light pulse passes; and (ii) a size of an aperture in an attachment that is attached to the treatment device.

In some embodiments the processing unit is further configured to identify when light pulses are applied to the skin of the body part. In these embodiments the processing unit is configured to identify when light pulses are applied to the skin of the body part by: processing the first measurement signal or received images or a video sequence to identify increased brightness due to a light pulse.

In some embodiments the processing unit is further configured to: generate an actuation signal to that is to cause a light source in the treatment device to generate a light pulse if the determined current treatment area does not overlap, or does not substantially overlap, with any of the previous treatment areas.

In some embodiments the apparatus is part of the treatment device. In alternative embodiments the apparatus is separate from the treatment device.

In some embodiments the apparatus comprises the first sensor. In alternative embodiments, the apparatus is separate from the first sensor.

According to a second aspect, there is provided a system, comprising: a treatment device comprising one or more light source(s) for performing a treatment operation on skin; a first sensor for outputting a first measurement signal comprising information about positions and/or movements of the treatment device over time; and an apparatus according to the first aspect or any embodiment thereof.

According to a third specific aspect, there is provided a computer-implemented method for providing feedback to a user on a treatment operation performed on a body part of a subject by a treatment device, wherein the treatment device is configured to apply light pulses to skin of the body part to perform the treatment operation, wherein a light pulse applied to the skin treats an area of the skin. The method comprises receiving a first measurement signal from a first sensor, the first measurement signal comprising information about positions and/or movements of the treatment device over time; for a light pulse previously applied by the treatment device to the body part during the treatment operation, processing the first measurement signal to estimate a previous treatment position as a position of the treatment device relative to the body part when the light pulse was generated; for the previously applied light pulse, estimating a previous treatment area for the light pulse corresponding to the area of skin of the body part that the light pulse was applied to when the treatment device was at the previous treatment position; processing the first measurement signal to estimate a current position of the treatment device relative to the body part; estimating a current treatment area corresponding to an area of skin that the treatment device would apply a light pulse to while in the current position; and generating a feedback control signal for a feedback unit, wherein the feedback control signal is configured to cause the feedback unit to generate feedback indicating whether the current treatment area corresponds, or substantially corresponds, to a previous treatment area.

In some embodiments the first sensor is one of: a movement sensor, an accelerometer, a gyroscope, a magnetometer, an air pressure sensor, and an imaging unit.

In some embodiments the sensor is an imaging unit, the first measurement signal is a plurality of images or a video sequence, and the plurality of images or the video sequence is processed to identify positions and/or movements of the treatment device over time. In these embodiments the method can further comprise: identifying skin features and/or body part features in the images or video sequence; monitoring movement of those skin features and/or body part features in the images or video sequence over time; and determining positions and/or movements of the treatment device relative to the body part from the movement of those skin features and/or body part features in the images or video sequence. In these embodiments the method can further comprise receiving a second measurement signal from a movement sensor, the second measurement signal comprising information about positions and/or movements of the treatment device over time; and the steps of processing the first measurement signal to estimate a previous treatment position and processing the first measurement signal to estimate a current position can comprise processing the first measurement signal and the second measurement signal to estimate the previous treatment positions and the current position.

In some embodiments the feedback control signal is a display control signal, and the feedback unit is a display unit. In these embodiments the display control signal can be configured to cause the display unit to generate a graphical display comprising graphical representations of the previous treatment areas and the current treatment area overlaid on the body part. In these embodiments the display unit may be a display screen and the display control signal may include a plurality of images or a video sequence of the body part on which the graphical representations are overlaid. In alternative embodiments, the display unit may be a projector and the display control signal may be configured to cause the projector to project the graphical representations onto the body part of the subject according to the estimated previous treatment areas and the current treatment area. In other alternative embodiments, the display unit may be configured to display the graphical representations as part of an augmented reality display of the body part.

In some embodiments the steps of estimating a previous treatment area and estimating a current treatment area can comprise estimating the previous treatment areas and estimating the current treatment area based on: (i) a size of an aperture in the treatment device through which the light pulse passes; and (ii) a size of an aperture in an attachment that is attached to the treatment device.

In some embodiments the method further comprises identifying when light pulses are applied to the skin of the body part. In these embodiments the step of identifying when light pulses are applied to the skin can comprise identifying when light pulses are applied to the skin of the body part by: processing the first measurement signal or received images or a video sequence to identify increased brightness due to a light pulse.

In some embodiments the method further comprises generating an actuation signal to that is to cause a light source in the treatment device to generate a light pulse if the determined current treatment area does not overlap, or does not substantially overlap, with any of the previous treatment areas.

According to a fourth aspect, there is provided a computer program product comprising a computer readable medium having computer readable code embodied therein, the computer readable code being configured such that, on execution by a suitable computer or processing unit, the computer or processing unit is caused to perform the method according to the third aspect or any embodiment thereof.

These and other aspects will be apparent from and elucidated with reference to the embodiment(s) described hereinafter.

### BRIEF DESCRIPTION OF THE DRAWINGS

Fig. 1 is an illustration of an exemplary treatment device with which the invention can be used;
Fig. 2 is a block diagram of an exemplary system comprising an imaging unit and an apparatus according to various embodiments;
Fig. 3 shows an infra-red image of an arm after a number of light pulses have been applied;
Fig. 4 is a flow chart illustrating an exemplary method according to the techniques described herein;
Fig. 5 shows two images obtained by an imaging unit;
Fig. 6 is an illustration of visual feedback that can be provided according to embodiments of the techniques described herein; and
Fig. 7 is an image showing exemplary visual feedback that can be provided according to embodiments of the techniques described herein.

### DETAILED DESCRIPTION OF THE EMBODIMENTS

As noted above, the techniques described herein can be used to determine and provide feedback on a treatment operation performed on a body part of a subject. In particular, the feedback can indicate whether a current position of the treatment device on the skin of the body part is skin that has already been treated (i.e. already had a light pulse applied). In particular embodiments, the feedback can be a graphical display that includes graphical representations of any areas of skin that have previously been treated and an indication of an area of skin that will be treated in the current position of the treatment device.

The techniques described herein can be implemented by the treatment device (e.g. by a processing unit in the treatment device), or implemented by a processing unit in a separate apparatus (e.g. a smartphone, tablet, smartwatch, laptop, computer, server, etc.). One or more sensors are required to provide information on positions (including orientation) and/or movements of the treatment device over time. In some embodiments, the one or more sensors includes one or more movement sensors, such as an accelerometer, gyroscope, etc. In these embodiments the one or more movement sensors may alternatively be referred to as an inertial measurement unit (IMU). In some embodiments, the one or more sensors includes an imaging unit (e.g. a camera) that obtains a plurality of images or a video sequence of an area of skin on a subject. In the case of a movement sensor (such as an accelerometer), the movement sensor may be part of the treatment device. In the case of an imaging unit, the imaging unit may be part of the treatment device, part of an apparatus separate from the treatment device that implements the techniques described herein, or separate from both the treatment device and any apparatus that implements the techniques described herein. It will be appreciated that in some embodiments the one or more sensors can include one or more movement sensors and an imaging unit.

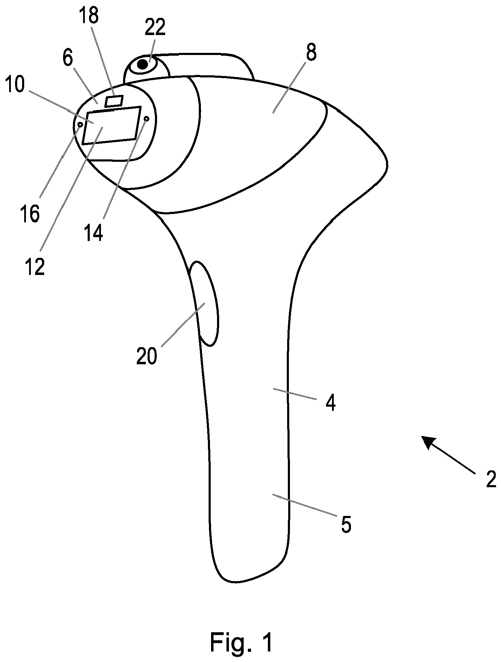

Fig. 1 is an illustration of an exemplary treatment device 2 that can be used to apply a light pulse to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. The treatment device 2 is for use on a body of a subject (e.g. a person or an animal), and is to be held in one or both hands of a user during use. The treatment device 2 is to perform some treatment operation to the skin or body of the subject using one or more light pulses when the treatment device 2 is in contact with, or close to, a body part of the subject. The treatment operation can be removal of unwanted hairs by laser and/or light therapies (known as a photoepilation treatment or Intense Pulsed Light, IPL, treatment). Alternatively the treatment operation can be laser and/or light therapies for reasons other than removal or preventing growth of hair, such as a dermatological (skin) treatment, treating acne, a phototherapy treatment for other types of skin condition, skin rejuvenation, skin tightening, or port-wine stain treatment.

As described herein, the treatment device 2 is operated or used by a 'user', and the treatment device 2 is used on a body of a 'subject'. In some cases the user and the subject is the same person, i.e. the treatment device 2 is held in a hand and used by a user on themselves (e.g. used on the skin on their leg). In other cases the user and the subject are different people, e.g. the treatment device 2 is held in a hand and used by a user on someone else. In either case, it is difficult for a user to achieve complete coverage of a body part and/or avoid over-treating certain areas of the body part since there are little or no user-perceptible changes to the skin on applying or shortly after applying a light pulse.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the treatment operation to be performed on the body or skin of the subject at the position that the head portion 6 is in contact with the body or skin.

The treatment device 2 is for performing a treatment operation using light pulses. Thus, in Fig. 1 the head portion 6 comprises an aperture 10 arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture 10 may be in the form of an opening at the head end 8 of the housing 4, or it may be in the form of a window (including a waveguide) that is transparent or semi-transparent to the light pulses (i.e. the light pulses can pass through the window).

In the exemplary embodiment shown in Fig. 1, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape.

Although not shown in Fig. 1, the treatment device 2 may have one or more removable attachments for the head portion 6 of the treatment device 2 that each includes an aperture 10. The attachments may be for use on different body parts, and have apertures 10 with different sizes. For example one attachment can be for use on a large body part such as the legs and therefore have a large aperture 10, whereas another attachment can be for use on the skin/hair above the upper lip and therefore have a small aperture 10.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any suitable type of light source, such as one or more light emitting diodes (LEDs), a (Xenon) flash lamp, a laser or lasers, etc. The light source(s) 12 can provide light pulses with spectral content in the 560-1200 nanometre (nm) range for a duration of around 2.5 milliseconds (ms), as these wavelengths heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). The intensity of the light pulse should be high enough to effect the treatment operation on the skin or body part adjacent the aperture 10.

The illustrated treatment device 2 also optionally includes two skin contact sensors 14, 16 positioned on or in the head portion 6 that are used to determine whether the head portion 6 is in contact with the skin. The skin contact sensors 14, 16 measure a parameter that is indicative of whether the head portion 6 is in contact with skin, and generate respective measurement signals that comprise a time-series of measurements of the parameter. The measurement signals can be processed to determine if the head portion 6 is in contact with skin. Typically a skin contact sensor is used in a treatment device 2, particularly a photoepilator, to make sure that the treatment device 2 is correctly in contact with skin before a light pulse is generated to avoid the light pulse being directed into the eyes of the user or subject.

In some embodiments the parameter can be capacitance, and so the skin contact sensors 14, 16 can measure capacitance via a respective pair of electrical contacts or electrodes on the surface of the head portion 6, with the measured capacitance being indicative of whether there is skin contact. In alternative embodiments, the parameter can be an intensity or level of light, and so the skin contact sensors 14, 16 can be light sensors that measure an intensity or level of light incident on the light sensor, with the measured intensity or level being indicative of whether there is skin contact (e.g. less/no light could indicate skin contact as the skin obscures the light sensors 14, 16, and vice versa). In other alternative embodiments, the parameter can be a measure of contact pressure, and so the skin contact sensors 14, 16 can measure contact pressure via respective pressure sensors or mechanical switches, with the measured contact pressure being indicative of whether there is skin contact.

The illustrated treatment device 2 also includes an optional skin tone sensor 18 positioned on or in the head portion 6 that can be used to determine a skin tone of the skin that the head portion 6 is in contact with. The skin tone sensor 18 can measure a parameter that is indicative of the skin tone of the skin, and generate a measurement signal that comprises a time-series of measurements of the parameter. The measurement signal can be processed to determine the skin tone of the skin that the head portion 6 is in contact with. Typically a skin tone sensor is used in a treatment device 2, particularly a photoepilator, to make sure that the light pulse has an intensity that is appropriate for the type of skin being treated, or even to prevent a light pulse being generated if the skin type is unsuitable for light pulses (e.g. darker skin which has a much higher melanin content).

In some embodiments the optional skin tone sensor 18 can be a light sensor and the parameter measured by the light sensor can be an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

As noted above, one or more sensors are required to provide information on positions (including orientations) and/or movements of the treatment device over time so that suitable feedback can be determined, and in some embodiments the one or more sensors can be part of or be in the treatment device 2. In the case of the sensor(s) being one or more movement sensors, such as an accelerometer, gyroscope, etc., the one or more movement sensors can be part of, e.g. inside, the treatment device 2 (and as such are not shown in Fig. 1). In embodiments where the one or more sensors includes an imaging unit (e.g. a camera), the imaging unit may be on or in the treatment device 2. As shown in Fig. 1, an imaging unit 22 may be arranged on the housing 4 of the treatment device 2 so that it is able to obtain images or a video sequence of skin or a body part that is in front of the treatment device 2. That is, the imaging unit 22 is arranged on the housing 4 of the treatment device 2 so that it obtains images or a video sequence from a direction that is generally parallel to the direction in which light pulses are emitted by the light source(s) 12 through the aperture 10. In some implementations, the imaging unit 22 is arranged on the housing 4 such that, when the head portion 6 is placed into contact with the subject so that a light pulse can be applied to the part of the skin visible to the light source(s) 12 through the aperture 10, the imaging unit 22 is not able to view the part of the skin in contact with the aperture 10 and head end 6. In embodiments where the imaging unit 22 is arranged on or in the housing 4 of the treatment device 2, the imaging unit 22 is in a fixed arrangement with respect to the rest of the treatment device 2.

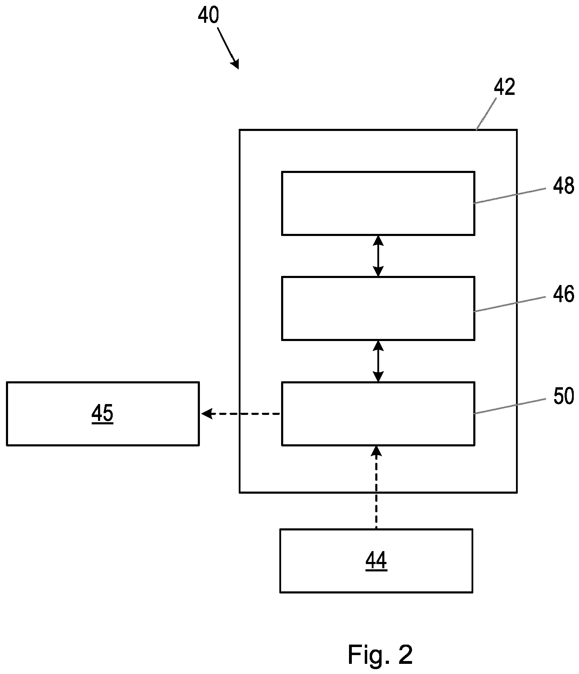

Fig. 2 is a block diagram of an exemplary system 40 comprising an apparatus 42 for providing feedback on areas of skin that have and have not been treated with a light pulse. The system 40 also comprises one or more sensors 44 for providing a measurement signal comprising information about positions and/or movements of the treatment device 2 over time, and a feedback unit 45 for providing feedback to the user and/or subject on areas of skin that have/have not been treated with a light pulse. In some implementations the treatment device 2 can be considered part of the system 40, although the treatment device 2 is not shown in Fig. 2. As noted above, in some embodiments the apparatus 42 can be a separate device to the treatment device 2, and thus the apparatus 42 may be in the form of an electronic device, such as a smart phone, smart watch, tablet, personal digital assistant (PDA), laptop, desktop computer, remote server, smart mirror, etc. In other embodiments, the apparatus 42, and particularly the functionality according to the invention provided by the apparatus 42, is part of the treatment device 2.

As noted above, the one or more sensors 44 can be one or more movement sensors 44, such as an accelerometer, a gyroscope, an air pressure sensor (that can be used for measuring altitude changes, a magnetometer, etc., that measure the movements of the treatment device 2 and output a measurement signal representing those movements. The one or more movement sensors 44 may be part of or in the treatment device 2. In the case of the one or more movement sensors 44 comprising an accelerometer, the accelerometer 44 can generate a measurement signal that contains a plurality of acceleration measurement samples representing the movements of the treatment device 2 at a plurality of time instants. The accelerometer 44 may be an accelerometer that measures accelerations in three dimensions, and the measurement signal generated by the accelerometer 44 can include respective measurement signals representing the accelerations in each of the three dimensions. For example, the accelerometer 44 can output respective measurement signals for each of an x-axis, y-axis and z-axis of a Cartesian coordinate system.

The one or more sensors 44 can also include or alternatively be an imaging unit 44 (e.g. a camera or an event camera) that obtains a plurality of images or a video sequence. An event camera is an imaging sensor in which each pixel of the sensor independently indicates changes in brightness as they occur. The plurality of images (including images formed from brightness indications from an event camera) or video sequence are output as a measurement signal, and the measurement signal (images/video sequence) can be processed using imaging processing techniques to identify movements of the treatment device 2. In some embodiments, the imaging unit 44 is attached to or part of the treatment device 2, in which case the images/video sequence can be processed to extract the movements of the treatment device 2 based on movements of objects (e.g. body parts or skin) visible in the images/video sequence. In other embodiments the imaging unit 44 can be separate from the treatment device 2, e.g. an imaging unit 4 in the apparatus 44, or in a separate device such as a smart phone, smart watch, tablet, personal digital assistant (PDA), laptop, desktop computer, smart mirror, etc. In this case, the imaging unit 44 may be able to observe both the treatment device 2 and the body part from a distance. An imaging unit 44 may include any suitable components for capturing an image, for example a charge-coupled device (CCD) and one or more lenses and/or mirrors. In some embodiments, the imaging unit 44 is a camera, such as a digital camera, or an event camera. As noted above, in some embodiments, the one or more sensors 44 can comprise one or more movement sensors and an imaging unit.

The apparatus 42 comprises a processing unit 46 that generally controls the operation of the apparatus 42 and enables the apparatus 42 to perform the method and techniques described herein. The processing unit 44 is to receive a measurement signal from a sensor 44, process the measurement signal to determine the feedback to be provided about the treatment operation, and provide a feedback control signal to the feedback unit 45. Thus the processing unit 46 can be configured to receive the measurement signal from the one or more sensors 44 either directly in embodiments where the sensor(s) 44 are part of the apparatus 42, or via another component in embodiments where the sensor(s) 44 are separate from the apparatus 42. In either case, the processing unit 46 can include or comprise one or more input ports or wires for receiving the measurement signal. The processing unit 46 can also include or comprise one or more output ports or wires for outputting the feedback control signal.

The processing unit 46 can be implemented in numerous ways, with software and/or hardware, to perform the various functions described herein. The processing unit 46 may comprise one or more microprocessors or digital signal processors (DSPs) that may be programmed using software or computer program code to perform the required functions and/or to control components of the processing unit 46 to effect the required functions. The processing unit 46 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor) and/or hardware specifically designed for simultaneous localisation and mapping (SLAM) techniques.

The processing unit 46 can comprise or be associated with a memory unit 48. The memory unit 48 can store data, information and/or signals (including image(s)) for use by the processing unit 46 in controlling the operation of the apparatus 42 and/or in executing or performing the methods described herein. In some implementations the memory unit 48 stores computer-readable code that can be executed by the processing unit 46 so that the processing unit 46 performs one or more functions, including the methods described herein. In particular embodiments, the program code can be in the form of an application for a smart phone, tablet, laptop, computer or server. The memory unit 48 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment shown in Fig. 2, as the apparatus 42 is shown as being separate from the sensor(s) 44, the apparatus 42 also includes interface circuitry 50 to enable the apparatus 42 to receive the measurement signal(s) from the sensor(s) 44. The interface circuitry 50 in the apparatus 42 enables a data connection to and/or data exchange with other devices, including any one or more of the sensor(s) 44, the treatment device 2, servers and databases. The connection to the sensor(s) 44 (or any electronic device, such as treatment device 2) may be direct or indirect (e.g. via the Internet), and thus the interface circuitry 50 can enable a connection between the apparatus 42 and a network, or directly between the apparatus 42 and another device (such as sensor(s) 44 and/or treatment device 2), via any desirable wired or wireless communication protocol. For example, the interface circuitry 50 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 50 (and thus apparatus 42) may include one or more suitable antennas for transmitting/receiving over a transmission medium (e.g. the air). Alternatively, in the case of a wireless connection, the interface circuitry 50 may include means (e.g. a connector or plug) to enable the interface circuitry 50 to be connected to one or more suitable antennas external to the apparatus 42 for transmitting/receiving over a transmission medium (e.g. the air). The interface circuitry 50 is connected to the processing unit 46.

Although not shown in Fig. 2, the apparatus 42 may comprise one or more user interface components that includes one or more components that enables a user of apparatus 42 to input information, data and/or commands into the apparatus 42, and/or enables the apparatus 42 to output information or data to the user of the apparatus 42. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

The feedback unit 45 is for generating feedback indicating whether the current treatment area corresponds, or substantially corresponds, to a previous treatment area. The feedback may be any of, or any combination of, visual, audible and tactile. Thus the feedback unit 45 may comprise any one or more output component(s), including but not limited to a display unit (such as a display screen, a display screen/transparent panel used for providing an augmented reality (AR) display or a projector, such as a pico-projector or micro-electromechanical system (MEMS) pico-projector), one or more lights or light elements, one or more loudspeakers, and one or more vibrating elements. In a preferred embodiment, the feedback unit 45 is a display unit and the feedback is a graphical display comprising graphical representations of areas of skin that have previously been treated with a light pulse, and an area that would be treated with a light pulse according to the current location of the treatment device 2.

In some embodiments, the feedback unit 45 is attached to or part of the treatment device 2. In other embodiments, the feedback unit 45 is part of the apparatus 42. In these embodiments the feedback unit 45 may be part of, or make use of, any of the output components of user interface components of the apparatus 42. In further embodiments, the feedback unit 45 can be separate from the treatment device 2 and the apparatus 42, for example in a separate device such as a smart phone, smart watch, tablet, personal digital assistant (PDA), laptop, desktop computer, smart mirror, etc.

It will be appreciated that a practical implementation of an apparatus 42 may include additional components to those shown in Fig. 2. For example the apparatus 42 may also include a power supply, such as a battery, or components for enabling the apparatus 42 to be connected to a mains power supply.

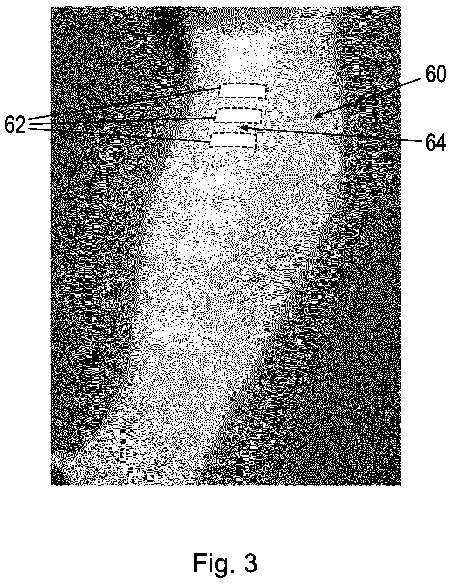

As discussed in the Background section, in a typical light-based treatment the user of the treatment device 2 must repeatedly manually position the treatment device 2 on the skin and trigger a light pulse. However, there are little or no user-perceptible changes to the skin or hairs on applying a light pulse or shortly after applying a light pulse, so it is difficult for a user to achieve complete coverage of a body part and/or avoid over-treating certain areas of the body part by repeating a light pulse on an area of skin that has already been treated. To illustrate a typical treatment coverage obtained without any feedback being provided to a user of the treatment device 2, Fig. 3 shows an infra-red image of an arm 60 after a number of light pulses have been applied. This infra-red image was obtained using a thermal camera. It will be appreciated that this image is included for ease of illustrating the problems addressed by the disclosed techniques, and the treatment device 2, apparatus 42 and system 40 do not include a thermal (infra-red) camera. In the image in Fig. 3, the brightness of a part of the image is dependent on the temperature of that part, with brighter (whiter) parts corresponding to higher temperatures and darker (blacker) parts corresponding to lower temperatures. It can be seen that there are a number of bright areas 62 (only some of which are labelled in Fig. 3) on the arm 60, which correspond to areas of higher temperature. These areas have a higher temperature due to a light pulse having recently been applied by a treatment device 2. A light pulse will cause a small (temporary) increase in the temperature of the part of the skin that the light pulse was applied to. It can be seen in Fig. 3 that many parts of the arm 60 have not recently been treated by a light pulse, and specifically there are gaps (labelled 64) between the bright areas 62. However, there is unlikely to be a visible change to the skin caused by the light pulses, so the user of the treatment device 2 will not be able to see which parts of the arm 60 have had light pulses applied. This illustrates the need for feedback for the user of the treatment device 2 so that they are able to identify which part or parts of the body part have already been treated with a light pulse.

Briefly, the techniques described herein allow for the identification or differentiation between areas of skin that light pulses have been applied to and areas that light pulses have not been applied to by tracking the position of the treatment device 2 relative to the body part when light pulses are applied, and tracking the current position of the treatment device 2 relative to those positions at which light pulses have been applied. A control signal for a feedback unit 45 (such as a display unit) can be generated that will cause the feedback unit 45 to provide feedback on whether the current treatment area (i.e. where the treatment device 2 is currently positioned) corresponds, or substantially corresponds, to a previous treatment area. Thus the feedback enables the user to better determine where on the body part the next light pulse should be applied.

In embodiments where the feedback is visual feedback, the visual feedback may be a graphical display comprising graphical representations of the previous treatment area(s) and the current treatment area overlaid on the body part. In this way the user of the treatment device 2 can easily see which parts of the body part have been treated and which parts still need to be treated.

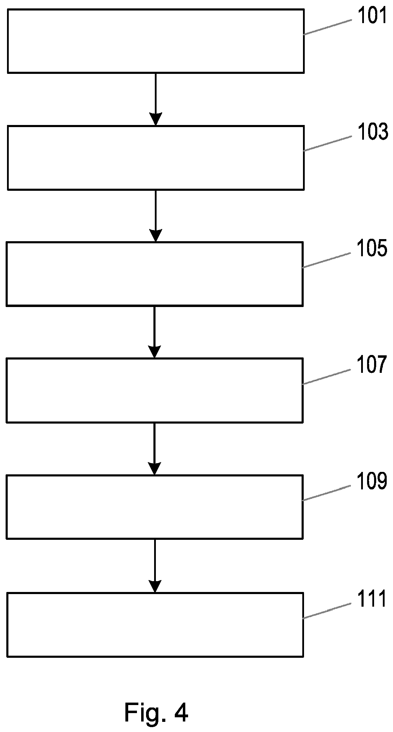

The flow chart in Fig. 4 illustrates an exemplary method according to the techniques described herein. One or more of the steps of the method can be performed by the processing unit 46 in the apparatus 42, in conjunction with any of the memory unit 48, interface circuitry 50 and user interface as appropriate. The processing unit 46 may perform the one or more steps in response to executing computer program code, that can be stored on a computer readable medium, such as, for example, the memory unit 48.

In step 101, a first measurement signal is received from sensor 44. The first measurement signal comprises information about positions and/or movements of the treatment device 2 over time. As noted above, the first measurement signal may be a signal from a movement sensor 44, or the first measurement signal may be a signal representing a plurality of images or a video sequence. In some embodiments, step 101 may comprise receiving a first measurement signal from a first sensor 44, e.g. a movement sensor 44, and a measurement signal from another sensor 44, e.g. an imaging unit.

In step 101 the first measurement signal can be received directly from the sensor 44, for example in real-time or near real-time as the measurement signal is generated by the sensor 44. Alternatively, the first measurement signal may have been previously generated by the sensor 44 and stored for subsequent analysis, for example in the memory unit 48, in a memory unit associated with the treatment device 2 or sensor 44, or in a remote database, in which case step 101 can comprise the processing unit 46 obtaining or retrieving the first measurement signal from the storage location (e.g. from memory unit 48, etc.). In these alternative embodiments, to provide the feedback to the user at a suitable time to enable the user to complete the treatment operation the first measurement signal should be obtained or retrieved shortly (e.g. no longer than a few seconds) after the first measurement signal is generated.

In step 103, for a light pulse previously applied by the treatment device 2 to the body part during the treatment operation, the processing unit 46 processes the first measurement signal to estimate the position of the treatment device 2 relative to the body part when this light pulse was generated or applied. This estimated position is referred to as a 'previous treatment position'.

In embodiments where the sensor 44 is one or more movement sensors, such as an accelerometer, the first measurement signal is a movement measurement signal and step 103 comprises processing the movement measurement signal to identify the movements and positions of the treatment device 2 relative to the body part. Techniques for processing measurements from movement sensors to determine movements and positions of the device in which the movement sensor(s) 44 are housed are known in the art, and will not be described further herein.

In embodiments where the sensor 44 is an imaging unit and the first measurement signal is a plurality of images or a video sequence, step 103 comprises processing the images or video sequence to identify the movements and positions of the treatment device 2 relative to the body part. In embodiments where the imaging unit 44 is attached to the treatment device 2, the processing unit 46 can process the images or video sequence to identify skin features and/or body part features in the images or video sequence, and monitor the movement of those skin features and/or body part features in the images or video sequence. The determined movement of those skin features and/or body part features in the images or video sequence corresponds to the movements of the treatment device 2 relative to the body part. The imaging unit 44 is fixed in or on the treatment device 2, and the relationship between the aperture 10 and the field of view of the imaging unit 44 will be known, which enables the processing unit 46 to determine the previous treatment position, i.e. the position of the treatment device 2 relative to the body part when a light pulse was applied. Techniques for processing the images or video sequence to identify skin features and/or body part features are generally known in the art of imaging processing, although some further details are provided below.

In alternative embodiments where the imaging unit 44 is separate from the treatment device 2 and is able to observe both the treatment device 2 and the body part from a distance, in step 103 the processing unit 46 can process the images or video sequence to identify the treatment device 2 and skin features and/or body part features, and monitor their relative positions over time. Techniques for processing the images or video sequence to identify a treatment device 2 and skin features and/or body part features are generally known in the art of imaging processing, although some further details are provided below.

In embodiments where the one or more sensors 44 comprises one or more movement sensors and an imaging unit, step 103 can comprise processing a first measurement signal from one of the movement sensor(s) and the imaging unit along with a second measurement signal from the other one of the movement sensor(s) and the imaging unit to estimate the position of the treatment device 2 relative to the body part.

In some embodiments, for example in the above embodiments where the one or more sensors 44 comprises one or more movement sensors and an imaging unit, step 103 can comprise using Simultaneous Localisation And Mapping (SLAM) techniques to determine the position of the treatment device 2 relative to the body part.

In step 103, having processed the first measurement signal to estimate the movements and/or positions of the treatment device 2 relative to the body part over time, a previous treatment position is determined as the estimated position of the treatment device 2 relative to the body part at the time that the light pulse was generated or applied.

In step 105, for the light pulse evaluated in step 103, the processing unit 46 estimates the area of skin of the body part that the light pulse was applied to. This estimated area is referred to as the 'previous treatment area' for the light pulse applied when the treatment device 2 was at the previous treatment position. The previous treatment position will generally have a known relationship between the estimated position and the aperture 10 of the treatment device 2. This enables step 105 to determine the appropriate area of the skin on the body part that the light pulse was applied to. For example, if previous treatment positions are estimated as the geometric centre of the aperture 10, then the previous treatment area can be centred on the relevant previous treatment position. The orientation of the treatment device 2 when the light pulse was applied is used to determine the orientation of the previous treatment area on the skin. Referring to Fig. 3, a previous treatment area estimated or determined in step 105 should generally correspond to a bright area 62.

The size of the previous treatment area will depend on the size of the aperture 10 in the treatment device 2. In some embodiments the size of the aperture 10 is fixed or predetermined, and the processing unit 46 can make use of information on the size of the aperture 10 (e.g. the dimensions of the aperture 10), and optionally other information that may affect the size of the area of skin that the light pulse is applied to, such as relative positions of the aperture 10 and light source(s) 12, to determine the size of the previous treatment area. Alternatively the processing unit 46 can determine the size of the aperture 10 by analysing an image of the aperture 10 (e.g. if, before use, an image of the aperture 10 is obtained using an imaging unit).

As noted above, in some embodiments the treatment device 2 may have a plurality of different attachments that can be used for treating different parts of the body. These attachments may have different sized apertures 10 to change the size of the area of skin treated with each light pulse. In this case, when determining the size of the previous treatment area, the processing unit 46 can make use of information on the size of the aperture 10 (e.g. the dimensions of the aperture 10), and optionally other information such as relative positions of the aperture 10 and light source(s) 12), that was used when that light pulse was applied. In some embodiments, the attachment that is in use on the treatment device 2 can be detected by the apparatus 42, for example by processing one or more images or a video sequence of the treatment device 2 to identify the attachment on the treatment device 2. In one example, before use of the treatment device 2, an image of the aperture 10 is obtained an imaging unit that is separate from the treatment device 2, and the processing unit 46 can determine the size of the aperture 10 by analysing the image of the aperture 10. The size of the aperture 10 can be determined by extracting the dimensions of the window/aperture 10 using processing techniques such as edge detections and (rectangle) shape fitting. Alternatively the apparatus 42 can receive a signal indicating the attachment that is attached to the treatment device 2. The treatment device 2 can provide the signal in response to detecting the type of attachment that is attached to the treatment device 2. The treatment device 2 can detect the type of attachment in a number of different ways, e.g. automatically based on signals exchanged between the body of the treatment device 2 and the attachment, or based on a user setting input to the treatment device 2 indicating the type of attachment attached or the type of body part to be treated. As another option, different attachments may have apertures 10 with different colours, and the apparatus 42 can identify the attachment that is in use by observing the colour of the light pulse when it is applied to the body part. Once the attachment is identified or otherwise known, information on the size of the aperture 10 associated with the identified attachment is obtained, e.g. from memory unit 48, and used to determine the size of the previous treatment area.

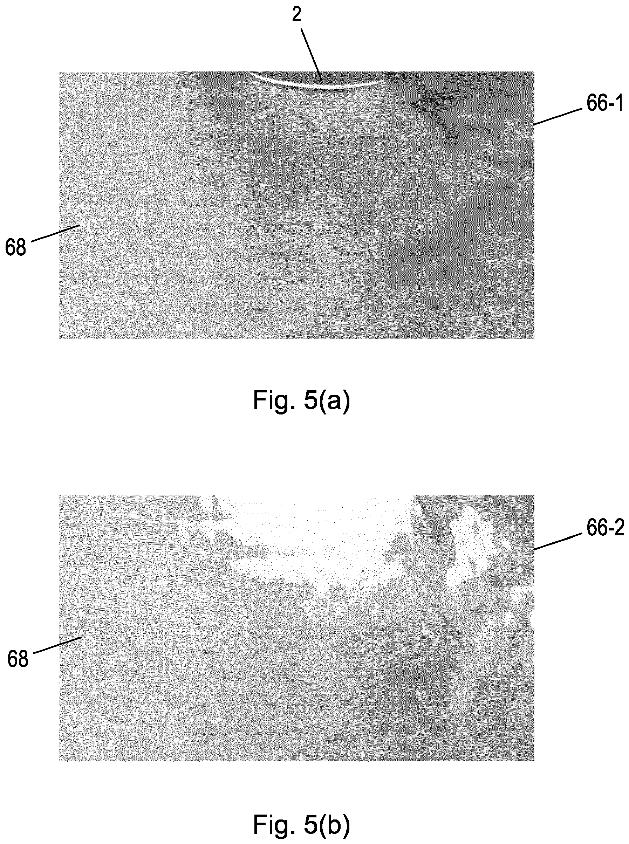

Steps 103 and 105 may be performed for a plurality of (or all) previous light pulses in the treatment operation. In preferred embodiments, as the feedback is to be provided to the user during the treatment operation to enable the user to determine where to position the treatment device 2 for the next light pulse, steps 103 and 105 are performed when, or shortly after, a light pulse is applied to the body part. As such, steps 103 and 105 may be performed in response to determining that a light pulse has been applied by the treatment device 2 to the body part. The processing unit 46 may determine that a light pulse has been applied to the body part in one (or more) of a number of different ways. In embodiments where the apparatus 42 is part of the treatment device 2, the processing unit 46 can receive a signal from the light source(s) 12, or from control circuitry for the light source(s) 12, indicating that a light pulse has been generated. Alternatively, where the apparatus 42 is separate from the treatment device 2, the processing unit 46 can receive a signal from the treatment device 2 indicating that a light pulse has been generated. As another alternative, where the sensor 44 is an imaging unit, or an imaging unit is otherwise provided in the system 40, the images or video sequence from the imaging unit can be processed to identify a light pulse. For example, Fig. 5 shows two images 66 of a body part 68 obtained by an imaging unit. In the image 66 (labelled 66-1) in Fig. 5(a), part of the treatment device 2 is visible, along with the skin of the body part 68. When this image was obtained, the treatment device 2 was not applying a light pulse to the body part 68. When the image 66 (labelled 66-2) in Fig. 5(b) was obtained, the treatment device 2 was applying a light pulse to the body part 68, and this is visible by the brighter top part of the image 66-2. Thus, in embodiments the processing unit 46 can be configured to process images or a video sequence to identify the occurrence of a light pulse. The occurrence of a light pulse can be identified by computing an average brightness for each image (or for series of images), and comparing the average brightnesses to identify if there are any image(s) that are significantly brighter (e.g. brighter by more than a threshold amount). Alternatively the average brightness of each image or series of images can be compared to a threshold amount to determine if the brightness is consistent with a light pulse having been applied. In some embodiments, the threshold amount can be adjusted based on the environmental lighting conditions. These embodiments have the benefit that the apparatus 42 does not need to be communicatively connected to the treatment device 2, and the apparatus 42 can be used with a conventional treatment device 2.

In step 107 the processing unit 46 processes the first measurement signal to estimate a current position of the treatment device 2 relative to the body part. The processing in step 107 can be the same as used in step 103 by the processing unit 46 to estimate the position of the treatment device 2 relative to the body part when a light pulse was generated or applied.

In step 109, the processing unit 46 estimates the area of skin of the body part that a light pulse would be applied to in the current position of the treatment device 2. This estimated area is referred to as the 'current treatment area'. The processing in step 109 can be the same as used in step 105 by the processing unit 46 to estimate the area of skin of the body part that a previous light pulse was applied to. As in step 105, in step 109 the size of the aperture 10 can be taken into account when estimating the size of the current treatment area.

In step 111, the processing unit 46 generates a feedback control signal for the feedback unit 45. The feedback control signal is generated so that it causes the feedback unit 45 to generate feedback indicating whether the current treatment area corresponds, or substantially corresponds, to a previous treatment area. The current treatment area corresponding to, or substantially corresponding to, a previous treatment area, may be when at least X% of the current treatment area overlaps with one or more previous treatment areas. X may be, for example, 20% (i.e. the current treatment area substantially corresponds to a previous treatment area when at least 20% of the current treatment area overlaps with one or more previous treatment areas), 50%, or a value in the range 20%-50%. In some embodiments, X may be a small value (e.g. in the range of 1%-5%) so as to prevent substantially any overlap between the current treatment area and any previous treatment area.

The method may further comprise providing the generated feedback control signal to the feedback unit 45 so that the feedback is provided to the user. As noted above, the feedback unit 45 may be configured to provide any combination of visual, audible and tactile feedback to indicate whether or not the treatment device 2 is at a position where a light pulse has previously been applied. The user of the treatment device 2 would be able to use these indications to determine whether to trigger a light pulse at the current position of the treatment device 2, which can help the user to improve the coverage of the treatment operation over the skin of the subject.

As an example, the feedback control signal could cause a red light (e.g. on the treatment device 2) to be illuminated if it is determined that the current treatment area overlaps with, or overlaps with too much of, a previous treatment area. Likewise the feedback control signal could cause a green light (e.g. on the treatment device 2) to be illuminated if it is determined that the treatment area does not overlap with, or does not overlap with too much of, a previous treatment area. As another example, the red/green light can be replaced with (or accompanied by) haptic feedback (e.g. vibrations of the treatment device 2) to indicate whether or not the current treatment area overlaps with, or overlaps with too much of, a previous treatment area. As another example, the red/green light can be replaced with (or accompanied by) audible feedback (e.g. a noise, audible message, etc.) to indicate whether or not the current treatment area overlaps with, or overlaps with too much of, a previous treatment area.

In a preferred embodiment, the feedback unit 45 is a display unit, such as a display screen, a transparent panel used for providing an AR display or a projector. In these embodiments, the feedback signal is a display control signal, that causes the display unit to generate a graphical display comprising graphical representations of the previous treatment areas and the current treatment area overlaid on the body part.

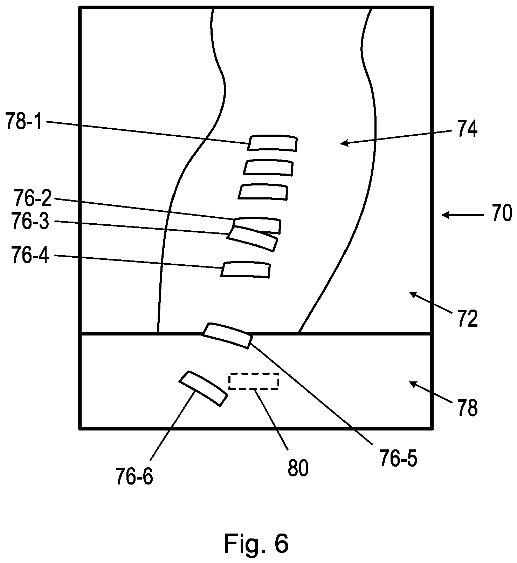

Fig. 6 is an illustration of visual feedback that can be provided by a display unit according to embodiments of the techniques described herein. In these embodiments, the visual feedback 70 includes a first portion 72 that is an image or a (live) video sequence of the body part 74 obtained using an imaging unit (which can be the sensor 44 used to determine the position and movements of the treatment device 2, or a separate imaging unit in the event that the one or more sensors 44 are movement sensors). Alternatively the first portion 72 can include a graphical representation on the body part 74. In Fig. 6 the body part 74 is a forearm of a subject. In the first portion 72 one or more graphical representations 76 of the previous treatment areas are overlaid on the image or video sequence of the body part 74. The previous treatment areas 76 are to be overlaid on the relevant positions on the body part 74 where those light pulses were applied. Thus, in generating the display control signal, the processing unit 46 determines where in the visual feedback 70 the previous treatment areas 76 should be located based on the estimated current position of the treatment device 2 relative to each of the estimated previous treatment positions. Several of the previous treatment areas 76 are individually labelled in Fig. 6, in particular previous treatment areas 76-1, previous treatment areas 76-2 and 76-3 (which partially overlap with each other) and previous treatment areas 76-4.

The visual feedback 70 in Fig. 6 also includes a second portion 76. In the case of an imaging unit that is located on the treatment device 2 (e.g. as shown in Fig. 1), when the treatment device 2 is in contact with skin, the imaging unit will typically not be able to view that particular part of the skin (and which corresponds to the current treatment area), and possibly some neighbouring skin depending on the field of view of the imaging unit, including whether a part of the treatment device 2 obscures the view of the imaging unit. The second portion 76 of the visual feedback 70 is used to display a graphical representation 80 of the current treatment region, along with graphical representations 76 of any previous treatment areas that are within the obscured area represented by the second portion 78. Thus, previous treatment area 76-5 is shown on the border between the first portion 72 and the second portion 78 (i.e. the previous treatment area 76-5 is partly in the first portion 72 and partly in the second portion 78). A previous treatment area 76-6 is shown that is completely in the second portion 78.

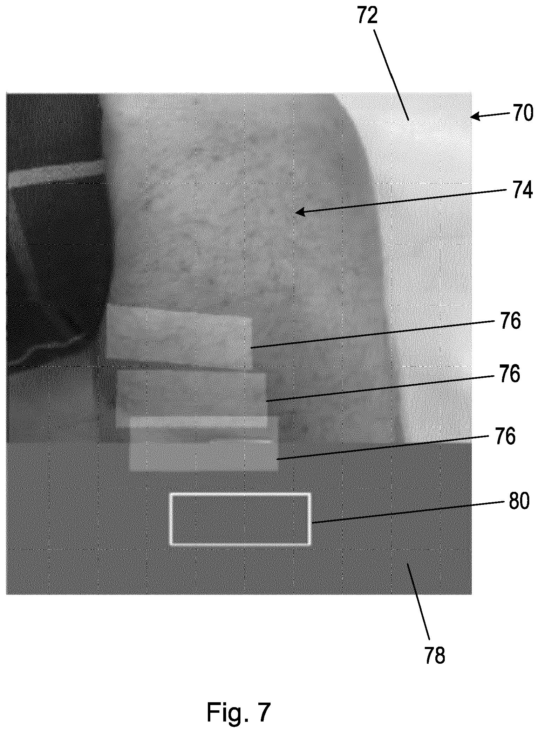

Fig. 7 shows exemplary visual feedback 70 that can be provided according to embodiments of the techniques described herein. The image in Fig. 7 provides feedback in line with the embodiment shown in Fig. 6, and thus Fig. 7 shows visual feedback 7 having a first portion 72 (a video sequence of the arm 74), several previous treatment areas 76 on the arm 74 in the first portion 72, and a second portion 78 showing a graphical representation 80 of the current treatment area.

In embodiments where the display unit is a projector, rather than display the graphical representations of the previous treatment areas and the current treatment area on an image or video sequence of the body part, the projector can project the graphical representations directly on to the body part in the appropriate positions. In this way the user is able to see the previously treated areas directly on the body part, which may make it easier for them to determine where the next light pulse should be applied.

In embodiments where the display unit is a transparent panel used for providing an AR display, the transparent panel can be positioned in the line of sight of the user, so that it is directly between the user and the body part being treated, enabling the user to view the body part through the transparent panel. In some embodiments the transparent panel is one or both lenses in a pair of glasses. The graphical representations of the previous treatment areas and the current treatment area can be projected or displayed on the transparent panel, so that they are overlaid on the correct positions of the body part from the point of view of the user. In this way the user is presented with an AR view of the body part that can enable them to more easily see the previously treated areas on the body part.

In embodiments where feedback is to be provided during use of the treatment device 2 (e.g. as the treatment device 2 is moved over the body part and light pulse(s) are applied), steps 101-111 can be performed generally continuously. That is, in step 101 a measurement signal can be received generally continuously (e.g. according to a sampling rate of the sensor 44, or according to a transmission rate (e.g. packet rate) from the sensor 44 to the apparatus 42), and steps 103-111 can be performed generally continuously to update the feedback to be provided to the user.

In embodiments where the sensor 44 is an imaging unit that is attached to the treatment device 2 and the processing unit 46 processes the images or video sequence to identify skin features and/or body part features in the images or video sequence in steps 103 and 105, the processing unit 46 can use one or more of a number of different techniques to process the images or video sequence. In some embodiments, skin features can be detected using feature detection algorithms such as Shi-Tomasi corner detection methods and/or scale-invariant feature transform (SIFT) methods. Alternatively, AI-based feature detection techniques that are optimised for skin features can be used. To estimate the movement of the treatment device 2 over time, the position of the detected skin features in the images or video sequence can be tracked over time by matching skin features between sequential images or sequential video frames. The movement of the treatment device 2 relative to the body part can be determined by calculating the homography between two sets of features of two sequential images or video frames. A homography determines the relationship between two imaging unit positions and orientations when viewing the same planar surface.

Over time, there will be a drift in the computation of the position of the current treatment position due to the accumulation of errors in the measurements and/or processing techniques. To reduce or avoid this drift, the detecting and movement tracking of skin features can be extended beyond two images or video frames so that skin features are tracked over multiple images or video frames.

Depending on the body part shown in the image(s), image processing techniques suitable for tracking position of skin features on non-planar surfaces, such as Simultaneous Localisation And Mapping (SLAM), can be used for the homography computation.

Features or objects visible in the background of the images or video sequence may induce errors in tracking the position of the skin feature or treatment device 2. To reduce these errors, the image(s) can be processed to identify and segment the skin area from the surrounding background. The parts of the image(s) relating to the background area are discarded and/or otherwise not used for skin feature tracking.

Fast movement of the treatment device 2 or imaging unit 44 may cause blurring of the obtained images and a reduction in the number of skin features that can be detected. This can cause a failure to determine and track the position of the treatment device 2. Image blurring can be reduced by using an imaging unit 44 a faster/higher frame rate and lower exposure times.

In some embodiments, feature tracking failure and/or drift error can be recovered by using loop closure. This can reset the drift in position by recognising a skin region based on the skin features that has been previously identified and that had moved outside the view of the imaging unit 44. Thus, the apparatus 42 can maintain information on the skin features identified in the images or video sequence, and use this information when skin features are identified in the next image or video sequence to determine if the skin area has previously been identified and has previously been treated with a light pulse.

In some embodiments, the treatment device 2 may be configured to automatically trigger a light pulse if the current treatment position of the treatment device 2 does not correspond, or substantially correspond, to a previous treatment area. In this case the apparatus 42 can be configured to automatically trigger the treatment device 2 to generate a light pulse. In some embodiments, one or more further conditions may be applied for triggering the light pulse, such as the treatment device 2 is in contact with skin, and/or the tone of the skin the treatment device 2 is in contact with is suitable to receive a light pulse. If a light pulse can be applied at the current position of the treatment device 2, the processing unit 46 can determine an actuation signal to actuate the light source(s) 12, or that causes the treatment device 2 to actuate the light source(s) 12.

In embodiments where one or more images are obtained in which the light pulse is visible, the processing unit 46 can be further configured to process the image(s) to determine the brightness and/or colour of the light pulses. The processing unit 46 can compare the determined brightnesses and/or colours of the light pulses to each other to determine if the brightness and/or colour of the light pulse is changing over time. Any changes in the brightness and/or colour of the light pulse over time (e.g. a decrease in brightness), can provide an indication of the health and/or decay of the light source(s) 12. If the brightness and/or colour meets a threshold criterion, an indication may be provided to the user that the light source(s) 12 may need to be replaced (if the light source(s) 12 are replaceable), or the treatment device 2 itself needs to be replaced.

In some embodiments, the feedback provided to the user can include feedback indicating whether the current treatment area corresponds, or substantially corresponds, to a previous treatment area, including those in a previous treatment operation. In these embodiments, following the method set out above to estimate the previous treatment positions and previous treatment areas for the light pulses applied during a treatment operation, information on these previous treatment positions and previous treatment areas is stored, for example in the memory unit 48. During the next treatment operation, this information is retrieved and used to form the feedback control signal in the next treatment operation. For example the feedback can indicate previous treatment areas from the previous treatment operation, and previous treatment areas from the current treatment operation.

Therefore there is provided methods and apparatus to determine feedback on a treatment operation performed on a body part of a subject to a user of the treatment device.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for use with a treatment device for providing feedback to a user on a treatment operation performed on a body part of a subject, wherein the treatment device is configured to apply light pulses to skin of the body part to perform the treatment operation, wherein a light pulse applied to the skin treats an area of the skin, the apparatus comprising a processing unit configured to:
receive a first measurement signal from a first sensor, the first measurement signal comprising information about positions and/or movements of the treatment device over time;
for a light pulse previously applied by the treatment device to the body part during the treatment operation, process the first measurement signal to estimate a previous treatment position as a position of the treatment device relative to the body part when the light pulse was generated;
for the previously applied light pulse, estimate a previous treatment area for the light pulse corresponding to the area of skin of the body part that the light pulse was applied to when the treatment device was at the previous treatment position;
process the first measurement signal to estimate a current position of the treatment device relative to the body part;
estimate a current treatment area corresponding to an area of skin that the treatment device would apply a light pulse to while in the current position; and
generate a feedback control signal for a feedback unit, wherein the feedback control signal is configured to cause the feedback unit to generate feedback indicating whether the current treatment area corresponds, or substantially corresponds, to a previous treatment area.

2. An apparatus as claimed in claim 1, wherein the sensor is an imaging unit, the first measurement signal is a plurality of images or a video sequence, and the plurality of images or the video sequence is processed to identify positions and/or movements of the treatment device over time.

3. An apparatus as claimed in claim 2, wherein the processing unit is configured to:
identify skin features and/or body part features in the images or video sequence;
monitor movement of those skin features and/or body part features in the images or video sequence over time; and
determine positions and/or movements of the treatment device relative to the body part from the movement of those skin features and/or body part features in the images or video sequence.

4. An apparatus as claimed in claim 2 or 3, wherein the processing unit is further configured to:
receive a second measurement signal from a movement sensor, the second measurement signal comprising information about positions and/or movements of the treatment device over time; and
wherein the processing unit is configured to process the first measurement signal and the second measurement signal to estimate the previous treatment positions and the current position.

5. An apparatus as claimed in any of claims 1-4, wherein the feedback control signal is a display control signal, and the feedback unit is a display unit.

6. An apparatus as claimed in claim 5, wherein the display control signal is configured to cause the display unit to generate a graphical display comprising graphical representations of the previous treatment areas and the current treatment area overlaid on the body part.

7. An apparatus as claimed in claim 6, wherein any one of:
the display unit is a display screen and the display control signal includes a plurality of images or a video sequence of the body part on which the graphical representations are overlaid;
the display unit is a projector and the display control signal is configured to cause the projector to project the graphical representations onto the body part of the subject according to the estimated previous treatment areas and the current treatment area; and
the display unit is configured to display the graphical representations as part of an augmented reality display of the body part.

8. An apparatus as claimed in any of claims 1-9, wherein the processing unit is configured to estimate the previous treatment areas and estimate the current treatment area based on: (i) a size of an aperture in the treatment device through which the light pulse passes; and (ii) a size of an aperture in an attachment that is attached to the treatment device.

9. An apparatus as claimed in any of claims 1-8, wherein the processing unit is further configured to:
generate an actuation signal to that is to cause a light source in the treatment device to generate a light pulse if the determined current treatment area does not overlap, or does not substantially overlap, with any of the previous treatment areas.

10. A system, comprising:
a treatment device comprising one or more light source(s) for performing a treatment operation on skin;
a first sensor for outputting a first measurement signal comprising information about positions and/or movements of the treatment device over time; and
an apparatus as claimed in any of claims 1-9.

11. A computer-implemented method for providing feedback to a user on a treatment operation performed on a body part of a subject by a treatment device, wherein the treatment device is configured to apply light pulses to skin of the body part to perform the treatment operation, wherein a light pulse applied to the skin treats an area of the skin, the method comprising:
receiving a first measurement signal from a first sensor, the first measurement signal comprising information about positions and/or movements of the treatment device over time;
for a light pulse previously applied by the treatment device to the body part during the treatment operation, processing the first measurement signal to estimate a previous treatment position as a position of the treatment device relative to the body part when the light pulse was generated;
for the previously applied light pulse, estimating a previous treatment area for the light pulse corresponding to the area of skin of the body part that the light pulse was applied to when the treatment device was at the previous treatment position;
processing the first measurement signal to estimate a current position of the treatment device relative to the body part;
estimating a current treatment area corresponding to an area of skin that the treatment device would apply a light pulse to while in the current position; and
generating a feedback control signal for a feedback unit, wherein the feedback control signal is configured to cause the feedback unit to generate feedback indicating whether the current treatment area corresponds, or substantially corresponds, to a previous treatment area.

12. A method as claimed in claim 11, wherein the sensor is an imaging unit, the first measurement signal is a plurality of images or a video sequence, and the plurality of images or the video sequence is processed to identify positions and/or movements of the treatment device over time.

13. A method as claimed in claim 12, wherein the method further comprises:
identifying skin features and/or body part features in the images or video sequence;
monitoring movement of those skin features and/or body part features in the images or video sequence over time; and
determining positions and/or movements of the treatment device relative to the body part from the movement of those skin features and/or body part features in the images or video sequence.

14. A method as claimed in claim 12 or 13, wherein the method further comprises:
receiving a second measurement signal from a movement sensor, the second measurement signal comprising information about positions and/or movements of the treatment device over time; and
wherein the step of processing the first measurement signal to estimate a previous treatment position and processing the first measurement signal to estimate a current position of the treatment device comprises processing the first measurement signal and the second measurement signal to estimate the previous treatment positions and the current position.

15. A computer program product comprising a computer readable medium having computer readable code embodied therein, the computer readable code being configured such that, on execution by a suitable computer or processing unit, the computer or processing unit is caused to perform the method of any of claims 11-14.
